# FASCICULE DE BREVET EUROPEEN

(11) **EP 1 297 104 B1**
(45) Date de publication et mention de la délivrance du brevet: **30.04.2008**
(21) Numéro de dépôt: 01984105.5
(22) Date de dépôt: 03.07.2001
(51) Int. Cl.: C12M 1/00

(54) **PROCEDE POUR AMELIORER LE TRANSFERT DANS UNE CHAMBRE DE BIOREACTION**
VERFAHREN ZUR VERBESSERUNG DES TRANSFERS IN EINER BIOLOGISCHEN REAKTIONSKAMMER
METHOD FOR IMPROVING TRANSFER IN A BIOLOGICAL REACTION CHAMBER

(30) Priorité: 03.07.2000 FR 0008587
(43) Date de publication de la demande: 02.04.2003
(73) Titulaire: INSTITUT FRANCAIS DE RECHERCHE POUR L'EXPLOITATION DE LA MER (IFREMER), 92138 Issy-les-Moulineaux Cedex (FR)
(72) Inventeur: MULLER-FEUGA, Arnaud, F-81110 Sorèze (FR); LEGRAND, Jack, F-44600 Saint-Nazaire (FR); PRUVOST, Jérémy, F-85220 Saint-Reverend (FR); LEGENTILHOMME, Patrick, F-44530 Dreffeac (FR); LE GUEDES, Roland, F-29460 L'Hopital-Camfrout (FR)
(74) Mandataire: Intes, Didier Gérard André
(86) Numéro de dépôt international: PCT/FR2001/002123
(87) Numéro de publication internationale: WO 2002/002734

(56) Documents cités:
- EP-A- 0 900 766
- WO-A-96/03494
- GB-A- 1 509 630
- US-A- 5 958 761

## Description

L'invention concerne le domaine de la culture en milieu réactionnel liquide de micro-organismes, ou de cellules isolées de macro-organismes végétaux ou animaux.

Elle concerne plus précisément un procédé pour améliorer le rendement d'un bioréacteur comportant au moins une chambre de bioréaction annulaire qui est délimitée par deux parois cylindriques coaxiales dont l'une au moins, dite paroi d'échange, permet un transfert de matières gazeuses ou liquides ou le passage de la lumière, et dans laquelle circule axialement un milieu réactionnel liquide contenant en suspension une culture de micro-organismes ou de cellules isolées de macro-organismes végétaux ou animaux, procédé selon lequel on soumet la culture à une réaction biosynthétique activée par la matière ou la lumière passant à travers ladite paroi d'échange.

L'invention se rapporte aussi bien à la culture de micro-organismes ou cellules isolées phototrophes, qu'à la culture de micro-organismes ou cellules isolées hétérotrophes.

Dans la culture de micro-organismes phototrophes, on apporte un rayonnement de lumière visible ainsi que du gaz carbonique qui est consommé au cours de la réaction de photosynthèse avec production d'oxygène. L'oxygène doit être éliminé car il est toxique au-delà d'une certaine concentration.

Dans la culture de micro-organismes hétérotrophes, on apporte de l'oxygène. L'oxygène est consommé, et il y a une production de gaz carbonique qui doit être éliminé car il présente une toxicité au-delà d'une certaine concentration.

FR-A 2 762 326 décrit un photobioréacteur pour la culture de micro-organismes photoautotrophes comportant une pluralité de chambres annulaires horizontales dont la paroi interne est transparente et entoure une chambre de lumière. Le milieu réactionnel est introduit axialement dans la chambre annulaire où il est soumis à un mouvement turbulent.

Lorsqu'un liquide est introduit axialement dans une chambre annulaire, les molécules restent sensiblement toujours à la même distance de l'axe de la chambre. Si le mouvement est turbulent, les molécules subissent de faibles déplacements radiaux autour d'une position moyenne. On en déduit que la culture située au voisinage de la paroi interne est fortement éclairée, tandis que la culture disposée au voisinage de la paroi externe est peu éclairée. Une très forte turbulence entraînerait peut être un renouvellement de la culture au voisinage de la paroi interne. Mais, les micro-organismes sont sensibles au cisaillement et une grande turbulence est néfaste à la production.

Dans FR A 2 762 326, on règle l'absorbance de la culture en ajoutant au milieu réactionnel des particules transparentes ou réfléchissantes ayant une densité sensiblement égale à celle du milieu réactionnel, le pourcentage volumique des particules dans le milieu réactionnel étant fonction de l'espèce de micro-organismes cultivés, de l'épaisseur de la chambre et de la concentration finale souhaitée de la culture dans le milieu réactionnel.

Cette disposition permet de faire en sorte que tous les micro-organismes soient éclairés au cours de leur transfert dans la chambre annulaire, quel que soit le type de micro-organismes. Mais elle ne permet pas de poursuivre une croissance à l'obscurité après que les micro-organismes aient été éclairés pendant une durée Tₗᵤₘ.

Le but de l'invention est d'améliorer le rendement de ce type de photobioréacteur, en créant dans la chambre un écoulement qui favorise le renouvellement de la culture au voisinage de la paroi éclairée afin que les micro-organismes ayant été éclairés poursuivent leur croissance lorsqu'ils rentrent dans des zones non éclairées.

Ainsi pour un débit donné du milieu réactionnel, la croissance des micro-organismes sera supérieure à celle obtenue par le procédé connu.

Le procédé selon l'invention peut également s'appliquer à des micro-organismes ou cellules isolées, autres que les micro-organismes phototrophes.

En particulier, lorsque la bioréaction est obtenue par la consommation d'un gaz (gaz carbonique ou oxygène) et production d'un autre gaz à éliminer (oxygène ou gaz carbonique), la paroi interne de la chambre de bioréaction peut être constituée par une membrane semi-perméable hydrophobe qui retient le milieu réactionnel dans la chambre et laisse passer l'un des gaz. Dans le cas d'une alimentation de gaz, ce dernier est sous pression dans le tube intérieur de la chambre (source). Dans le cas d'une élimination de gaz, le tube intérieur (puits) est en dépression.

Il est à noter que la paroi d'échange de gaz peut être la paroi extérieure de la chambre de bioréaction, cette dernière est alors disposée dans un conduit de plus grand diamètre servant à amener un gaz de préférence sous pression (source). Les deux parois de la chambre de bioréaction peuvent être semi-perméables et hydrophobes, la paroi interne formant un puits en dépression pour l'élimination du gaz toxique, et la paroi externe étant connectée à une source de gaz à consommer lors de la bioréaction. De même, l'une des parois peut être transparente pour permettre l'éclairage de la culture et l'autre paroi peut être semi-perméable et de préférence hydrophobe pour permettre l'apport d'un gaz ou l'élimination du gaz toxique produit pendant la photobioréaction.

Lorsque la bioréaction nécessite l'apport d'une solution nutritive minérale ou organique, la paroi interne de la chambre de bioréaction est constituée d'une membrane poreuse hydrophile et définit une source de solution nutritive sous pression.

Le procédé selon l'invention est caractérisé par le fait que l'on soumet le milieu réactionnel circulant dans ladite chambre de bioréaction à un écoulement primaire tourbillonnaire hélicoïdal autour de l'axe de la chambre, qui sous l'action de la force centrifuge crée des tourbillons secondaires rotatifs, afin de favoriser le renouvellement de la culture au voisinage de la paroi d'échange.

Avantageusement, on soumet le milieu réactionnel à un écoulement primaire tourbillonnaire hélicoïdal en introduisant le milieu réactionnel à l'une des extrémités de la chambre de bioréaction dans une direction sensiblement perpendiculaire à l'axe de la chambre et excentrée par rapport à cet axe.

De préférence, on introduit le milieu réactionnel dans la chambre de bioréaction par un conduit ayant un axe sensiblement perpendiculaire à l'axe de la chambre, et raccordé tangentiellement à la paroi externe de ladite chambre.

La dimension transversale interne du conduit dans la direction perpendiculaire à l'axe de la chambre de bioréaction est au plus égale à l'épaisseur radiale de ladite chambre.

D'autres avantages et caractéristiques de l'invention ressortiront à la lecture de la description suivante faite à titre d'exemple et en référence aux dessins annexés dans lesquels :
la figure 1 montre une chambre annulaire d'un photobioréacteur selon l'art antérieur ;
la figure 2 est un graphique montrant les déplacements radiaux des micro-organismes dans une chambre annulaire selon l'art antérieur dans laquelle le milieu de réaction entre dans une direction axiale disposée dans le plan de symétrie de la chambre ;
la figure 3 montre de manière schématique une chambre de bioréaction selon l'invention et le conduit d'amenée du milieu réactionnel ;
la figure 4 est une coupe radiale de la chambre de bioréaction par un plan passant par l'axe du conduit d'amenée du milieu réactionnel ;
la figure 5 montre de manière schématique la création des tourbillons secondaires rotatifs ; et
la figure 6 est un graphique montrant les déplacements radiaux des micro-organismes dans la chambre de bioréaction selon l'invention.

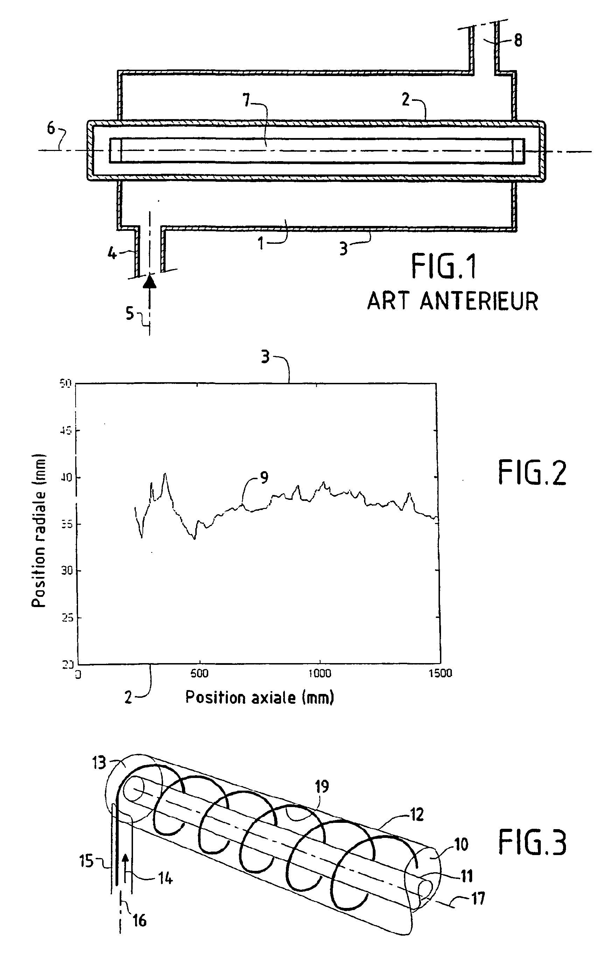

La figure 1 montre une chambre 1 d'un photobioréacteur selon FR A 762 326. Cette chambre annulaire est délimitée intérieurement par une paroi cylindrique transparente 2 et extérieurement par une deuxième paroi cylindrique 3. Le milieu réactionnel liquide est introduit à une extrémité de la chambre 1 par un conduit d'entrée 4 dont l'axe 5 est situé dans le plan passant par l'axe 6 de la chambre 1. Un tube luminescent 7 est disposé dans l'axe 6 de la paroi transparente 2. La chambre 1 a une longueur sensiblement égale à 1500 mm et une épaisseur voisine de 30 mm. Le milieu réactionnel s'écoule axialement dans la chambre 1 de manière turbulente et est évacué par un conduit de sortie 8 disposé à l'extrémité de la chambre 1 opposée au conduit d'entrée 4.

Le graphique de la figure 2 montre le rayon de la trajectoire 9 d'un micro-organisme dans la chambre 1. Le cheminement de ce micro-organisme subit de faibles déplacements radiaux au cours de son déplacement axial dans la chambre 1. Il en résulte qu'il n'y a pas de renouvellement de la culture au voisinage de la paroi transparente 2.

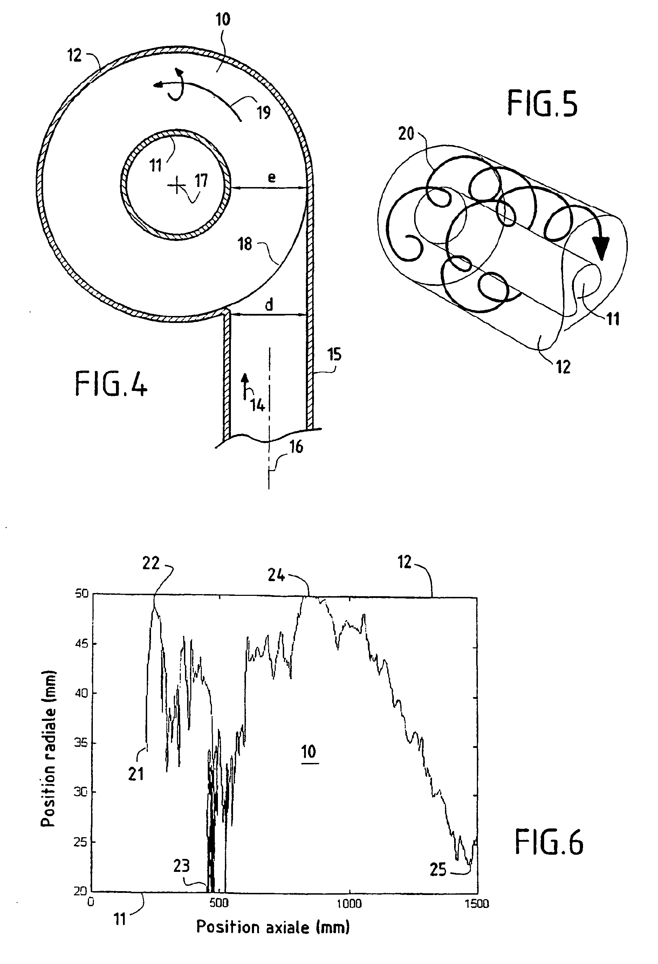

Les figures 3 et 4 montrent une chambre de bioréaction 10, de type annulaire délimitée par une paroi cylindrique interne 11 et une paroi cylindrique externe 12 reliées à l'une de leur extrémité au moins par un fond 13. La paroi interne 11 et la paroi externe 12 sont coaxiales, et au moins la paroi interne 11, dite paroi d'échange, permet le passage d'une matière gazeuse ou liquide ou le passage de la lumière, nécessaire à la bioréaction de micro-organismes ou de cellules isolées de macro-organismes végétaux ou animaux en suspension dans un milieu réactionnel 14, liquide qui circule dans la chambre annulaire 10. Le milieu réactionnel 14 est introduit à l'extrémité de la chambre 10, au voisinage du fond 13, au moyen d'un conduit 15, dont l'axe 16 est perpendiculaire à l'axe 17 de la chambre 10, et décentré par rapport à cet axe 17. Le conduit d'amenée 15 est raccordé tangentiellement à la paroi externe 12 de la chambre 10. Autrement dit, le conduit 15 présente une génératrice éloignée de l'axe 17 de la chambre 10 qui est tangente à la paroi externe 12. La section du conduit d'amenée 15 est circulaire ou curviligne, mais sa dimension d dans la direction perpendiculairement à l'axe 17 de la chambre 10 est au plus égale à l'espace radial e séparant les deux parois 11 et 12 de la chambre 10. Le cylindre de l'orifice 18 du conduit d'amenée 15 ne présente donc pas d'intersection avec la paroi cylindrique interne 10.

Le débit de milieu réactionnel liquide délivré par le conduit d'amenée 15 pénètre donc tangentiellement et sensiblement perpendiculairement à l'axe 17 à l'une des extrémités de la chambre annulaire 10. Du fait que le conduit d'amenée 15 est situé au voisinage immédiat du fond 13, le milieu réactionnel 14 circule dans la chambre 10 selon un écoulement primaire tourbillonnaire hélicoïdal autour de l'axe 17 de la chambre 10. L'axe hélicoïdal de cet écoulement primaire tourbillonnaire est représenté par la référence 19 sur la figure 3. En outre, du fait des forces centrifuges, le mouvement primaire crée des tourbillons secondaires 20 rotatifs autour de l'axe hélicoïdal du tourbillon primaire. L'écoulement tourbillonnaire 20 ainsi obtenu n'étant pas entretenu, a tendance à s'atténuer par diminution de la vitesse circonférentielle. Il reste cependant actif sur une distance de 1500 mm au moins.

L'axe 16 du conduit d'amenée 15 n'est pas obligatoirement rectiligne. Ce qui compte c'est que le milieu réactionnel 14 pénètre dans la chambre 11 selon une direction décentrée par rapport à l'axe 17 et sensiblement perpendiculaire à celui-ci et que le débit pénètre tangentiellement à la paroi externe 12, pour éviter des cisaillements et des recirculations dans cette zone d'entrée.

La section du conduit d'entrée 15 est inférieure à la section transversale de la chambre 10, afin que la vitesse axiale Va du milieu réactionnel dans la chambre soit inférieure à sa vitesse dans le conduit d'amenée Vc. Le facteur de vitesse VcNa est par exemple de 4 et peut être compris dans une large gamme, par exemple entre 2 et 10. Ce facteur de vitesse est choisi de telle sorte qu'il y ait un tourbillon 19 bien formé sur toute la longueur de la chambre 10, et que le renouvellement de la culture au niveau de la paroi interne 11 soit assurée.

La figure 6 montre le rayon de la trajectoire d'un micro-organisme dans la chambre 10, lorsque le milieu réactionnel est introduit ainsi que cela a été décrit ci-dessus. On constate qu'à partir du point d'entrée médian 21, le micro-organisme s'est rapproché de la paroi externe 12 au point 22, puis revient vers la paroi interne 11 au point 23, se rapproche de nouveau de la paroi externe 12 au point 24 pour revenir au voisinage de la paroi interne 11, au point 25, à l'autre extrémité de la chambre de bioréaction 10.

Il est à noter que les spécifications sur la longueur du conduit d'entrée 15 ne sont pas contraignantes, des écoulements primaires tourbillonnaires ayant été obtenus, avec des longueurs de conduit de 12 mm. Les spécifications sur l'orifice de sortie de la chambre 10 ne sont pas contraignantes. En particulier, la position tangentielle de conduit de sortie n'est pas requise.

Dans l'exemple décrit en détail ci-dessus et s'appliquant à un photobioréacteur, la paroi interne 11 est transparente et permet le passage d'une lumière émise par une source de lumière. Cette paroi peut être une paroi semi-perméable, hydrophobe, et la cavité interne de la paroi interne 11 peut contenir du gaz carbonique sous pression pour nourrir les micro-organismes ou peut être mise en dépression pour extraire du milieu réactionnel l'oxygène produit au cours de la bioréaction et qui est toxique au delà d'une certaine concentration.

Cette paroi 10 peut être poreuse et hydrophile. La cavité interne sert alors en tant que source de solution nutritive sous pression pour la culture de micro-organismes photo ou hétérotrophes.

## Revendications

1. Procédé pour améliorer le rendement d'un bioréacteur comportant au moins une chambre de bioréaction (10) annulaire qui est délimitée par deux parois cylindriques (11, 12) coaxiales dont l'une au moins, dite paroi de d'échange (11), permet un transfert de matières gazeuses ou liquides ou le passage de la lumière, et dans laquelle circule axialement un milieu réactionnel liquide (14) contenant en suspension une culture de micro-organismes ou de cellules isolées de macro-organismes végétaux ou animaux, procédé selon lequel on soumet la culture à une réaction biosynthétique activée par la matière ou la lumière passant à travers ladite paroi d'échange (11),
**caractérisé par le fait que** l'on soumet le milieu réactionnel (14) circulant dans ladite chambre de bioréaction (10) à un écoulement primaire (19) tourbillonnaire hélicoïdal autour de l'axe (17) de la chambre (10), qui sous l'action de la force centrifuge crée des tourbillons secondaires (20) rotatifs, afin de favoriser le renouvellement de la culture au voisinage de la paroi d'échange (11).

2. Procédé selon la revendication 1, **caractérisé par le fait que** l'on soumet le milieu réactionnel (14) à un écoulement primaire tourbillonnaire hélicoïdal en introduisant le milieu réactionnel à l'une des extrémités de la chambre de bioréaction (10) dans une direction sensiblement perpendiculaire à l'axe (17) de la chambre et excentrée par rapport à cet axe (17).

3. Procédé selon la revendication 2, **caractérisé par le fait que** l'on introduit le milieu réactionnel (14) dans la chambre de bioréaction (10) par un conduit (15) ayant un axe (16) sensiblement perpendiculaire à l'axe (17) de la chambre (10), et raccordé tangentiellement à la paroi externe (12) de ladite chambre (10).

4. Procédé selon la revendication 3, **caractérisé par le fait que** la dimension transversale interne (d) du conduit (15) dans la direction perpendiculaire à l'axe (17) de la chambre (10) de bioréaction est au plus égale à l'épaisseur (e) radiale de ladite chambre (10).

## Claims

1. A method of improving the yield of a biological reactor comprising at least one annular biological reaction chamber (10) defined by two coaxial cylindrical walls (11, 12), at least one of which is an exchange wall (11) allowing gaseous or liquid matter to be transferred or allowing light to pass, and in which there flows axially a liquid reaction medium (14) containing a culture of microorganisms or of isolated cells of vegetable or animal microorganisms in suspension, in which method the culture is subjected to a biosynthesis reaction activated by the matter or the light passing through said exchange wall (11),
the method being **characterized by** the fact that the reaction medium (14) flowing in said biological reaction chamber (10) is subjected to a primary turbulent flow (19) that is helical about the axis (17) of the chamber (10), which flow under the action of centrifugal force generates rotary secondary vortices (20), thereby encouraging the culture to be renewed in the vicinity of the exchange wall (11).

2. A method according to claim 1, **characterized by** the fact that the reaction medium (14) is subjected to a helical turbulent primary flow by introducing the reaction medium at one of the ends of the biological reaction chamber (10) in a direction that is substantially perpendicular to the axis (17) of the chamber and that is offset away from said axis (17).

3. A method according to claim 2, **characterized by** the fact that the reaction medium (14) is introduced into the biological reaction chamber (10) by means of a duct (15) having an axis (16) that is substantially perpendicular to the axis (17) of the chamber (10), and that is connected tangentially to the outer wall (12) of said chamber (10).

4. A method according to claim 3, **characterized by** the fact that the internal transverse dimension (d) of the duct (15) in the direction perpendicular to the axis (17) of the biological reaction chamber (10) is no greater than the radial thickness (e) of said chamber (10).

## Patentansprüche

1. Verfahren zur Verbesserung des Wirkungsgrades eines Bioreaktors, der wenigstens eine ringförmige Bioreaktionskammer (10) aufweist, welche durch zwei koaxiale zylindrische Wände (11, 12) begrenzt ist, von denen wenigstens eine, die sogenannte Austauschwand (11), einen Übergang gasförmiger oder flüssiger Stoffe oder den Durchgang des Lichts ermöglicht, und in welcher ein flüssiges Reaktionsmedium (14), das eine Kultur von Mikroorganismen oder von isolierten Zellen pflanzlicher oder tierischer Makroorganismen in Suspension enthält, axial zirkuliert, Verfahren, wonach die Kultur einer biosynthetischen Reaktion unterzogen wird, die durch den Stoff oder das Licht, der bzw. das die genannte Austauschwand (11) durchquert, aktiviert wird,
**dadurch gekennzeichnet, daß** das in der Bioreaktionskammer (10) zirkulierende Reaktionsmedium (14) einer primären spiralförmigen Wirbelströmung (19) um die Achse (17) der Kammer (10) unterworfen wird, die unter der Wirkung der Fliehkraft, sekundäre Drehwirbel (20) erzeugt, um die Erneuerung der Kultur in der Nähe der Austauschwand (11) zu begünstigen.

2. Verfahren nach Anspruch 1, **dadurch gekennzeichnet, daß** das Reaktionsmedium (14) einer primären spiralförmigen Wirbelströmung dadurch unterworfen wird, daß das Reaktionsmedium an einem der Enden der Bioreaktionskammer (10) in einer zur Achse (17) der Kammer im wesentlichen senkrechten und gegenüber dieser Achse (17) außermittigen Richtung eingeleitet wird.

3. Verfahren nach Anspruch 2, **dadurch gekennzeichnet, daß** das Reaktionsmedium (14) über eine Leitung (15), die eine zur Achse (17) der Kammer (10) im wesentlichen senkrechte Achse (16) aufweist und die an die Außenwand (12) der Kammer (10) tangential angeschlossen ist, in die Bioreaktionskammer (10) eingeleitet wird.

4. Verfahren nach Anspruch 3, **dadurch gekennzeichnet, daß** die innere Querabmessung (d) der Leitung (15) in der zur Achse (17) der Bioreaktionskammer (10) senkrechten Richtung höchstens gleich der radialen Dicke (e) der Kammer (10) ist.
